# EUROPÄISCHE PATENTSCHRIFT

(11) **EP 1 441 726 B1**
(45) Veröffentlichungstag und Bekanntmachung des Hinweises auf die Patenterteilung: **20.12.2006**
(21) Anmeldenummer: 02802623.5
(22) Anmeldetag: 10.10.2002
(51) Int. Cl.: A61K 31/425, A61K 31/445, C07D 275/02, C07D 417/12

(54) **DERIVATE DES PHENOXY-N-'4-(ISOTHIAZOLIDIN-1,1-DIOXID-2YL)PHENY]-VALERIAN-SÄUREAMIDS UND ANDERE VERBINDUNGEN ALS INHIBITOREN DES KOAGULATIONSFAKTORS XA ZUR BEHANDLUNG VON THROMBOEMBOLISCHEN ERKRANKUNGEN UND TUMOREN**
DERIVATIVES OF PHENOXY-N-'4-(ISOTHIAZOLIDIN-1,1-DIOXID-2YL)PHENY]-VALERIAN- ACID AMIDE AND OTHER COMPOUNDS AS INHIBITORS OF THE COAGULATION FACTOR XA IN THE TREATMENT OF THROMBOEMBOLIC DISEASES AND TUMORS
DERIVES D'AMIDE D'ACIDE PHENOXY-N-'4-(ISOTHIAZOLIDINE-1,1-DIOXYDE-2-YL)-PHENYL-VALERIANIQUE ET AUTRE COMPOSES SERVANT D'INHIBITEURS DU FACTEUR DE COAGULATION XA POUR LE TRAITEMENT DE TROUBLES THROMBOEMBOLIQUES ET DE TUMEURS

(30) Priorität: 09.11.2001 DE 10155075
(43) Veröffentlichungstag der Anmeldung: 04.08.2004
(73) Patentinhaber: Merck Patent GmbH, 64271 Darmstadt (DE)
(72) Erfinder: DORSCH, Dieter, 64372 Ober-Ramstadt (DE); CEZANNE, Bertram, 64546 Mörfelden-Walldorf (DE); TSAKLAKIDIS, Christos, 69469 Weinheim (DE); MEDERSKI, Werner, 64673 Zwingenberg (DE); GLEITZ, Johannes, 64293 Darmstadt (DE); BARNES, Christopher, 65812 Bad Soden (DE)
(86) Internationale Anmeldenummer: PCT/EP2002/011349
(87) Internationale Veröffentlichungsnummer: WO 2003/039543

(56) Entgegenhaltungen:
- EP-A- 0 352 613
- WO-A-00/55153
- WO-A-01/55146
- WO-A-01/85726
- WO-A-02/30930
- WO-A-94/04496
- WO-A-95/18797
- WO-A-99/06390
- WO-A-99/09027
- WO-A-99/26621
- WO-A-99/29346
- DE-A- 19 804 085
- DATABASE CA [Online] CHEMICAL ABSTRACTS SERVICE, COLUMBUS, OHIO, US; 1992 GROUTAS, W.C. ET AL: "1,2,5,-Thiadiazolidin-3-one 1,1 Dioxide: A powerful Scaffold for Probing the S' Subsites of (Chymo)trypsin-Like Serine Proteases" retrieved from STN, accession no. 2000:898004 Database accession no. 134:307088 XP002223273 & JOURNAL OF MEDICINAL CHEMISTRY (1992), 35(13), 2407-14 ,

## Beschreibung

Die Erfindung betrifft Verbindungen der Formel l 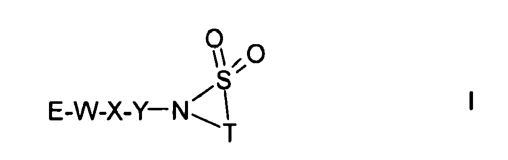 worin
- E: einfach durch R¹ substituiertes Phenyl oder Isochinolyl,
- R¹: CN, Amidino, Hal, NH₂, CH₂NH₂ oder 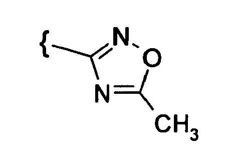
- W: OCHAr', OCHA, NHCHAr', NHCHA, NHCOOCHAr', NHCONHCHAr' oder Piperidin-1,2-diyl,
- Ar': unsubstituiertes oder ein- oder zweifach durch Hal, A oder CF₃ substituiertes Phenyl,
- A: Alkyl mit 1, 2, 3, 4, 5, 6, 7 oder 8 C-Atomen,
- X: CONH,
- Y: Ar-diyl,
- T: (CH₂)₃,
- Ar: unsubstituiertes oder ein- oder zweifach durch Hal, A oder CF₃ substituiertes Phenyl,
Hal F, Cl, Br oder I
bedeuten,
sowie ihre pharmazeutisch verwendbaren Salze, Solvate und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie Faktor Xa inhibierende Eigenschaften und können daher zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens eingesetzt werden.

Die erfindungsgemäßen Verbindungen der Formel I können weiterhin Inhibitoren der Gerinnungsfaktoren Faktor Vlla, Faktor lXa und Thrombin der Blutgerinnungskaskade sein.

Aromatische Amidinderivate mit antithrombotischer Wirkung sind z.B. aus der EP 0 540 051 B1, WO 98/28269, WO 00/71508, WO 00/71511, WO 00/71493, WO 00/71507, WO 00/71509, WO 00/71512, WO 00/71515 oder WO 00/71516 bekannt. Cyclische Guanidine zur Behandlung thromboembolischer Erkrankungen sind z.B. in der WO 97/08165 beschrieben. Aromatische Heterocyclen mit Faktor Xa inhibitorischer Aktivität sind z.B. aus der WO 96/10022 bekannt. Substituierte N-[(Aminoiminomethyl)-phenylalkyl]-azaheterocyclylamide als Faktor Xa Inhibitoren sind in WO 96/40679 beschrieben.

Andere gerinnungshemmende Faktor Xa Inhibitoren kennt man aus WO 01/55146 A1. In DE 198 04 085 A1 sind 5gliedrige heterocyclische kondensierte Benzoderivate mit anti-thrombotischer Wirkung beschrieben. In der WO 99/09027 sind (Hetero)Aryl-sulfonamidderivate als Faktor Xa Inhibitoren offenbart.

Der antithrombotische und antikoagulierende Effekt der erfindungsgemäßen Verbindungen wird auf die inhibierende Wirkung gegenüber der aktivierten Gerinnungsprotease, bekannt unter dem Namen Faktor Xa, oder auf die Hemmung anderer aktivierter Serinproteasen wie Faktor VIIa, Faktor IXa oder Thrombin zurückgeführt.

Faktor Xa ist eine der Proteasen, die in den komplexen Vorgang der Blutgerinnung involviert ist. Faktor Xa katalysiert die Umwandlung von Prothrombin in Thrombin. Thrombin spaltet Fibrinogen in Fibrinmonomere, die nach Quervernetzung elementar zur Thrombusbildung beitragen. Eine Aktivierung von Thrombin kann zum Auftreten von thromboembolischen Erkrankungen führen. Eine Hemmung von Thrombin kann jedoch die in die Thrombusbildung involvierte Fibrinbildung inhibieren.
Die Messung der Inhibierung von Thrombin kann z.B. nach der Methode von G. F. Cousins et al. in *Circulation* **1996**, *94*, 1705-1712 erfolgen.

Eine Inhibierung des Faktors Xa kann somit verhindern, daß Thrombin gebildet wird.
Die erfindungsgemäßen Verbindungen der Formel 1 sowie ihre Salze greifen durch Inhibierung des Faktors Xa in den Blutgerinnungsprozeß ein und hemmen so die Entstehung von Thromben.

Die Inhibierung des Faktors Xa durch die erfindungsgemäßen Verbindungen und die Messung der antikoagulierenden und antithrombotischen Aktivität kann nach üblichen in vitro- oder in vivo- Methoden ermittelt werden. Ein geeignetes Verfahren wird z.B. von J. Hauptmann et al. in *Thrombosis and Haemostasis* **1990**, *63,* 220-223 beschrieben.

Die Messung der Inhibierung von Faktor Xa kann z.B. nach der Methode von T. Hara et al. in *Thromb. Haemostas.* **1994,** *71*, 314-319 erfolgen.

Der Gerinnungsfaktor Vlla initiiert nach Bindung an Tissue Faktor den extrinsischen Teil der Gerinnungskaskade und trägt zur Aktivierung des Faktors X zu Faktor Xa bei. Eine Inhibierung von Faktor VIIa verhindert somit die Entstehung des Faktors Xa und damit eine nachfolgende Thrombinbildung.
Die Inhibierung des Faktors Vlla, durch die erfindungsgemäßen Verbindungen und die Messung der antikoagulierenden und antithrombotischen Aktivität kann nach üblichen in vitro- oder in vivo-Methoden ermittelt werden. Ein übliches Verfahren zur Messung der Inhibierung von Faktor VIIa wird z.B. von H. F. Ronning et al. in *Thrombosis Research* **1996**, *84*, 73-81 beschrieben.

Der Gerinnungsfaktor IXa wird in der intrinsischen Gerinnungskaskade generiert und ist ebenfalls an der Aktivierung von Faktor X zu Faktor Xa beteiligt. Eine Inhibierung von Faktor IXa kann daher auf andere Weise verhindern, daß Faktor Xa gebildet wird.
Die Inhibierung von Faktor IXa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Chang et al. in *Journal of Biological Chemistry* **1998***, 273*,12089-12094 beschrieben.

Die erfindungsgemäßen Verbindungen können weiterhin zur Behandlung von Tumoren, Tumorerkrankungen und/oder Tumormetastasen verwendet werden.
Ein Zusammenhang zwischen dem Tissuefaktor TF / Faktor VIIa und der Entwicklung verschiedener Krebsarten wurde von T.Taniguchi und N.R.Lemoine in Biomed. Health Res. (2000), 41 (Molecular Pathogenesis of Pancreatic Cancer), 57-59, aufgezeigt.
Die im nachfolgenden aufgeführten Publikationen beschreiben eine antitumorale Wirkung von TF-VH und Faktor Xa Inhibitoren bei verschiedenen Tumorarten:
K.M. Donnelly et al. in Thromb. Haemost. 1998; 79: 1041-1047;
E.G. Fischer et al. in J. Clin. Invest. 104: 1213-1221 (1999);
B.M. Mueller et al. in J. Clin. Invest. 101: 1372-1378 (1998);
M.E. Bromberg et al. in Thromb. Haemost. 1999; 82: 88-92

Die Verbindungen der Formel I können als Arzneimittelwirkstoffe in der Human- und Veterinärmedizin eingesetzt werden, insbesondere zur Behandlung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, venöse Thrombose, pulmonale Embolie, arterielle Thrombose, myocardiale Ischämie, instabile Angina und auf Thrombose basierender Schlaganfall.
Die erfindungsgemäßen Verbindungen werden auch zur Behandlung oder Prophylaxe von atherosklerotischen Erkrankungen wie koronarer arterieller Erkrankung, cerebraler arterieller Erkrankung oder peripherer arterieller Erkrankung eingesetzt.
Die Verbindungen werden auch in Kombination mit anderen Thrombolytika bei myocardialem Infarkt eingesetzt, ferner zur Prophylaxe zur Reocclusion nach Thrombolyse, percutaner transluminaler Angioplastie (PTCA) und koronaren Bypass-Operationen.

Die erfindungsgemäßen Verbindungen werden ferner verwendet zur Prävention von Rethrombose in der Mikrochirurgie, ferner als Antikoagulantien im Zusammenhang mit künstlichen Organen oder in der Hämodialyse.
Die Verbindungen finden ferner Verwendung bei der Reinigung von Kathetern und medizinischen Hilfsmitteln bei Patienten *in vivo,* oder als Antikoagulantien zur Konservierung von Blut, Plasma und anderen Blutprodukten *in vitro.* Die erfindungsgemäßen Verbindungen finden weiterhin Verwendung bei solchen Erkrankungen, bei denen die Blutkoagulation entscheidend zum Erkrankungsverlauf beiträgt oder eine Quelle der sekundären Pathologie darstellt, wie z.B. bei Krebs einschließlich Metastasis, entzündlichen Erkrankungen einschließlich Arthritis, sowie Diabetes.

Die erfindungsgemäßen Verbindungen finden weiterhin Verwendung zur Behandlung von Migräne (F.Morales-Asin et al., Headache, 40, 2000, 45-47).

Bei der Behandlung der beschriebenen Erkrankungen werden die erfindungsgemäßen Verbindungen auch in Kombination mit anderen thrombolytisch wirksamen Verbindungen eingesetzt, wie z.B. mit dem "tissue plasminogen activator" t-PA, modifiziertem t-PA, Streptokinase oder Urokinase. Die erfindungsgemäßen Verbindungen werden mit den anderen genannten Substanzen entweder gleichzeitig oder vorher oder nachher gegeben.
Besonders bevorzugt ist die gleichzeitige Gabe mit Aspirin, um ein Neuauftreten der Thrombenbildung zu verhindern.
Die erfindungsgemäßen Verbindungen werden auch verwendet in Kombination mit Blutplättchen-Glycoprotein-Rezeptor (IIb/IIIa)-Antagonisten, die die Blutplättchenaggregation inhibieren.

Gegenstand der Erfindung sind die Verbindungen der Formel I und ihre Salze sowie ein Verfahren zur Herstellung von Verbindungen der Formel 1 nach den Ansprüchen 1-13 sowie ihrer pharmazeutisch verwendbaren Salze, Solvate und Stereoisomere, dadurch gekennzeichnet, daß man
a) sie aus einem ihrer funktionellen Derivate durch Behandeln mit einem solvolysierenden und/oder hydrogenolysierenden Mittel in Freiheit setzt, indem man
   i) eine Amidinogruppe aus ihrem Oxadiazolderivat oder Oxazolidinonderivat durch Hydrogenolyse oder Solvolyse freisetzt,
   ii) eine konventionelle Aminoschutzgruppe durch Behandeln mit einem solvolysierenden oder hydrogenolysierenden Mittel durch Wasserstoff ersetzt oder eine durch eine konventionelle Schutzgruppe geschützte Aminogruppe in Freiheit setzt,
b) einen Rest E in einen anderen Rest E umwandelt, indem man
   i) eine Cyangruppe zu einer Amidinogruppe umsetzt,
   ii) eine Amidgruppe zu einer Aminoalkylgruppe reduziert,
   iii) eine Cyangruppe zu einer Aminoalkylgruppe reduziert,
c) zur Herstellung einer Verbindung der Formel I,
   worin X CONH bedeutet,
   eine Verbindung der Formel IV

   E-W-CO-L IV
worin
- L: Cl, Br, I oder eine freie oder reaktionsfähig funktionell abgewandelte OH-Gruppe bedeutet bedeutet
und E und W die in Anspruch 1 angegebenen Bedeutungen haben, mit der Maßgabe, daß falls eine weitere OH- und/oder Aminogruppe vorliegt, diese geschützt ist,
mit einer Verbindung der Formel V 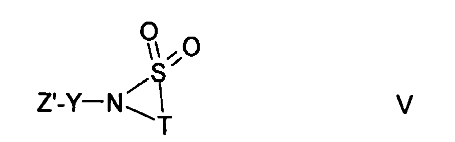 worin
- Z': NH₂ bedeutet
und Y und T die in Anspruch 1 angegebenen Bedeutungen haben, umsetzt,
und anschließend gegebenenfalls eine Schutzgruppe abspaltet,
und/oder
eine Base oder Säure der Formel l in eines ihrer Salze umwandelt.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaien versteht man mit z. B. Alkyl- oder Acylgruppen, Zuckern oder Oligopeptiden abgewandelte Verbindungen der Formel 1, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Gegenstand der Erfindung sind auch Mischungen der erfindungsgemäßen Verbindungen der Formel l, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Für alle Reste, die mehrfach auftreten, wie z.B. A, gilt, daß deren Bedeutungen unabhängig voneinander sind.
Vor- und nachstehend haben die Reste bzw. Parameter E, W, X, Y, T die bei der Formel I angegebenen Bedeutungen, falls nicht ausdrücklich etwas anderes angegeben ist.

Tritt in einer Verbindung ein- und derselbe Rest mehrfach auf, wie z.B. R², so sind dessen Bedeutungen unabhängig voneinander.

R¹ bedeutet vorzugsweise z.B. CN, Amidino, Hal, NH₂, CH₂NH₂ oder

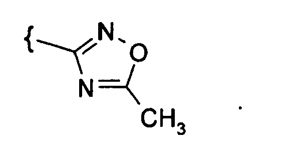

R¹ bedeutet ganz besonders bevorzugt Amidino, Hal, NH₂ oder CH₂NH₂.

E bedeutet vorzugsweise z.B. einen durch R¹ substituierten Phenyl- oder Isochinolinring.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2-, 2,3- oder 3,3-Dimethylbutyl, 1- oder 2-Ethylbutyl, 1-Ethyl-1-methylpropyl, 1-Ethyl-2-methylpropyl, 1,1,2-oder 1,2,2-Trimethylpropyl, weiter bevorzugt z.B. Trifluormethyl.
A bedeutet ganz besonders bevorzugt Alkyl mit 1-6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.
Hal bedeutet vorzugsweise F, Cl oder Br, aber auch I.
X bedeutet
ganz besonders bevorzugt CONH.
Y bedeutet vorzugsweise Ar-diyl, besonders bevorzugt unsubstituiertes oder einfach durch A oder F substituiertes 1,4-Phenylen. Y bedeutet insbesondere unsubstituiertes oder einfach durch Methyl, Ethyl oder Propyl substituiertes 1,3- oder 1,4-Phenylen.
Ar bedeutet ganz besonders bevorzugt unsubstituiertes oder ein- oder zweifach durch Hal, A oder CF₃ substituiertes Phenyl.
Ar' bedeutet ganz besonders bevorzugt unsubstituiertes oder ein- oder zweifach durch Hal, A oder CF₃ substituiertes Phenyl.
T bedeutet vorzugsweise (CH₂)₃.

Die Verbindungen der Formel I Können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis Ib ausgedrückt werden, die der Formel l entsprechen und worin die nicht näher bezeichneten Reste die bei der Formel I angegebene Bedeutung haben, worin jedoch
in la
- E: einfach durch R¹ substituiertes Phenyl oder Isochinolyl,
- R¹: CN, Amidino, Hal, NH₂, CH₂NH₂ oder 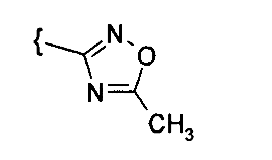
- W: OCHAr', OCHA, NHCHAr', NHCHA, NHCOOCHAr', NHCONHCHAr' oder Piperidin-1,2-diyl,
- Ar': unsubstituiertes oder ein- oder zweifach durch Hal, A oder CF₃ substituiertes Phenyl,
- A: Alkyl mit 1, 2, 3, 4, 5, 6, 7 oder 8 C-Atomen,
- X: CONH,
- Y: Ar-diyl,
- T: (CH₂)₃
bedeuten;
in lb
- E: einfach durch R¹ substituiertes Phenyl oder Isochinolyl,
- R¹: CN, Amidino, Hal, NH₂, CH₂NH₂ oder 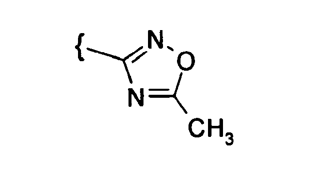
- W: OCHAr', OCHA, NHCHAr', NHCHA, NHCOOCHAr', NHCONHCHAr' oder Piperidin-1 ,2-diyl,
- Ar': unsubstituiertes oder ein- oder zweifach durch Hal, A oder CF₃ substituiertes Phenyl,
- A: Alkyl mit 1, 2, 3, 4, 5, 6, 7 oder 8 C-Atomen,
- X: CONH,
- Y: Ar-diyl,
- T: (CH₂)₃
- Ar: unsubstituiertes oder ein- oder zweifach durch Hal, A oder CF₃ substituiertes Phenyl
bedeuten;
sowie ihre pharmazeutisch verwendbaren Derivate, Solvate und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Ausgangsverbindungen der Formeln IV und V sind in der Regel bekannt. Sind sie neu, so können sie aber nach an sich bekannten Methoden hergestellt werden.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Verbindungen der Formel I können weiter vorzugsweise erhalten werden, indem man Verbindungen der Formel IV mit Verbindungen der Formel V umsetzt.
Die Umsetzung erfolgt in der Regel in einem inerten Lösungsmittel und unter Bedingungen wie oben angegeben.
In den Verbindungen der Formel IV bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6C-Atomen (bevorzugt Methylsulfonyloxy oder Trifluormethylsulfonyloxy) oder Arylsulfonyloxy mit 6-10C-Atomen (bevorzugt Phenyl- oder p-Tolylsulfonyloxy).
Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.
Aktivierte Ester werden zweckmäßig in situ gebildet, z. B. durch Zusatz von HOBt oder N-Hydroxysuccinimid.

Die Umsetzung erfolgt in der Regel in einem inerten Lösungsmittel, in Gegenwart eines säurebindenden Mittels vorzugsweise einer organischen Base wie DIPEA, Triethylamin, Dimethylanilin, Pyridin oder Chinolin oder eines Überschusses der Carboxykomponente der Formel IV.
Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.
Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.
Als inerte Lösungsmittel eignen sich die oben genannten.

Verbindungen der Formel I können ferner erhalten werden, indem man Verbindungen der Formel I aus einem ihrer funktionellen Derivate durch Behandeln mit einem solvolysierenden oder hydrogenolysierenden Mittel in Freiheit setzt.

Die Freisetzung der Amidinogruppe aus ihrem Oxadiazolderivat kann z.B. durch Behandeln mit Wasserstoff in Gegenwart eines Katalysators (z.B.
Raney-Nickel) abgespalten werden. Als Lösungsmittel eignen sich die nachfolgend .angegebenen, insbesondere Alkohole wie Methanol oder Ethanol, organische Säuren wie Essigsäure oder Propionsäure oder Mischungen daraus. Die Hydrogenolyse wird in der Regel bei Temperaturen zwischen etwa 0 und 100° und Drucken zwischen etwa 1 und 200 bar, bevorzugt bei 20-30° (Raumtemperatur) und 1-10 bar durchgeführt.

Die Einführung der Oxadiazolgruppe gelingt z.B. durch Umsetzung der Cyanverbindungen mit Hydroxylamin und Reaktion mit Phosgen, Dialkylacarbonat, Chlorameisensäureester, N,N'-Carbonyldiimidazol oder Acetanhydrid.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.
Bevorzugte Ausgangsstoffe sind auch die Oxadiazolderivate, die in die entsprechenden Amidinoverbindungen überführt werden können.

Es können auch mehrere - gleiche oder verschiedene - geschützte Amino-und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfembar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10C-Atomen. Beispiele für Hydroxyschutzgruppen sind u.a. Benzyl, 4-Methoxybenzyl, p-Nitrobenzoyl, p-Toluolsulfonyl, tert.-Butyl und Acetyl, wobei Benzyl und tert.-Butyl besonders bevorzugt sind.

Das In-Freiheit-Setzen der Verbindungen der Formel 1 aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfembare Schutzgruppen (z. B. CBZ, Benzyl oder die Freisetzung der Amidinogruppe aus ihrem Oxadiazolderivat)) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumforrniat (anstelle von Wasserstoff) an Pd/C in Methanol/DMF bei 20-30°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Trifluormethylbenzol, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Umwandlung einer Cyangruppe in eine Amidinogruppe erfolgt durch Umsetzung mit z.B. Hydroxylamin und anschließender Reduktion des N-Hydroxyamidins mit Wasserstoff in Anwesenheit eines Katalysators wie z.B. Pd/C.
Zur Herstellung eines Amidins der Formel I kann man an ein Nitril auch Ammoniak anlagern. Die Anlagerung erfolgt bevorzugt mehrstufig, indem man in an sich bekannter Weise a) das Nitril mit H₂S in ein Thioamid umwandelt, das mit einem Alkylierungsmittel, z.B. CH₃l, in den entsprechenden S-Alkyl-imidothioester übergeführt wird, welcher seinerseits mit NH₃ zum Amidin reagiert, b) das Nitril mit einem Alkohol, z.B. Ethanol in Gegenwart von HCl in den entsprechenden Imidoester umwandelt und diesen mit Ammoniak behandelt (Pinner-Synthese), oder c) das Nitril mit Lithium-bis-(trimethylsilyl)-amid umsetzt und das Produkt anschließend hydrolysiert.

Ester können z.B. mit Essigsäure oder mit NaOH oder KOH in Wasser, Wasser-THF oder Wasser-Dioxan bei Temperaturen zwischen 0 und 100° verseift werden.

Ferner kann man freie Aminogruppen in üblicher Weise mit einem Säurechlorid oder -anhydrid acylieren oder mit einem unsubstituierten oder substituierten Alkylhalogenid alkylieren, oder mit CH₃-C(=NH)-OEt umsetzen, zweckmäßig in einem inerten Lösungsmittel wie Dichlormethan oder THF und /oder in Gegenwart einer Base wie Triethylamin oder Pyridin bei Temperaturen zwischen -60 und +30°.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.
Auch physiologisch unbedenkliche organische Basen, wie z.B. Ethanolamin können verwendet werden.

Einige der Ausgangsverbindungen sind neu. Daher sind weiterhin Gegenstand der Erfindung die Zwischenverbindungen der Formel I-I 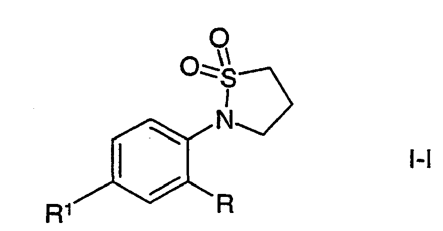 worin
- R¹: NO₂ oder NH₂
- R: Methyl, Chlor oder Trifluormethyl
bedeuten,
sowie deren Salze.

Die Herstellung erfolgt wie im Reaktionsschema des Beispiels 2 angegeben.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder oder auch als Nasenspray. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, Tumoren, Tumorerkrankungen und/oder Tumormetastasen verwendet werden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Gegenstand der Erfindung sind ferner Arzneimittel enthaltend mindestens eine Verbindung der Formel I und/oder ihre pharmazeutisch verwendbaren Salze, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Salze, Solvate und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Derivate, Solvate und Stereoisomere, einschließlich deren Mischungen in allen Verhältnissen,
und einer wirksamen Menge eines weiteren Arzneimittelwirkstoffs gelöst oder in lyophylisierter Form vorliegt.

Gegenstand der Erfindung ist ferner die Verwendung von Verbindungen der Formel 1 und/oder ihrer pharmazeutisch verwendbaren Salze, Solvate und Stereoisomere, einschließlich deren Mischungen in allen Verhältnissen,
zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen, in Kombination mit mindestens einem weiteren Arzneimittelwirkstoff.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
- Massenspektrometrie (MS):: El (Elektronenstoß-lonisation) M⁺
FAB (Fast Atom Bombardment) (M+H)⁺
ESI (Electrospray lonization) (M+H)⁺ (wenn
nichts anderes angegeben)

### Beispiel 1

Die Herstellung von 2-(3-Amidino-phenoxy)-*N*-[4-(isothiazolidin-1,1-dioxid-2-yl)-phenyl]-valeriansäureamid erfolgt wie in nachstehendem Schema angegeben:

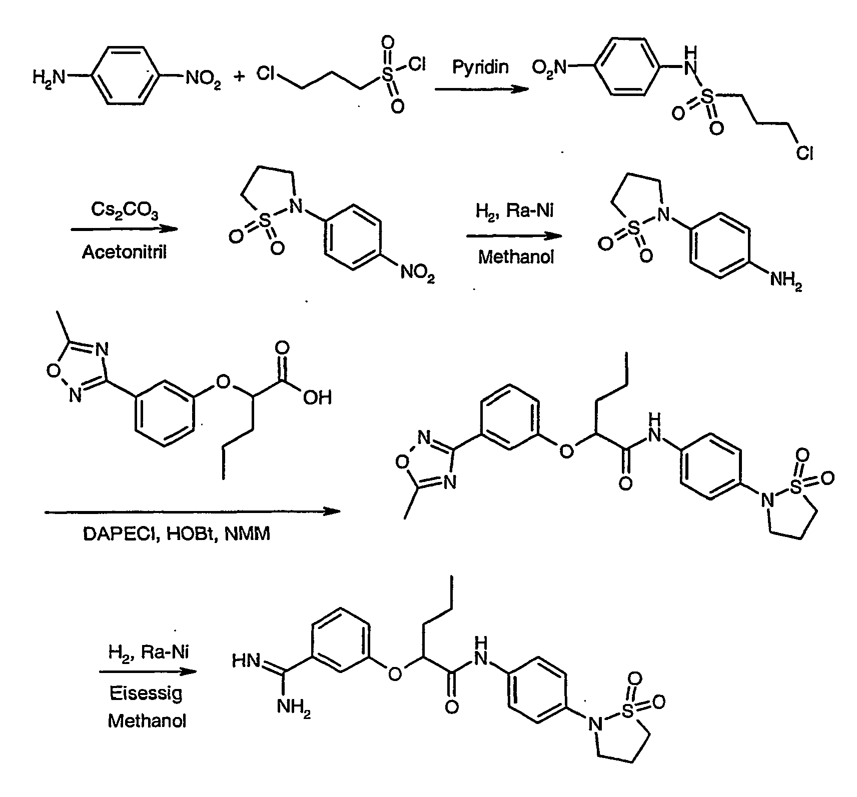

Eine Lösung von 5.00 g (36.2 mmol) 4-Nitroanilin und 6.41 g (36.2 mmol) 3-Chlorpropansulfonylchlorid in 20 ml Pyridin wird über Nacht bei Raumtemperatur gerührt. Dann wird das Reaktionsgemisch auf Eis gegossen. Der dabei ausgefallene Niederschlag wird abgesaugt und getrocknet: 3-Chlorpropan-1-sulfonsäure-(4-nitro-phenyl)-amid als gelblicher Feststoff; ESI 279.

Eine Lösung von 7.40 g (26.6 mmol) 3-Chlorpropan-1-sulfonsäure-(4-nitrophenyl)-amid in 150 ml Acetonitril wird mit 13.0 g (40.0 mmol) Caesiumcarbonat versetzt und 18 Stunden bei 70°C gerührt. Das Reaktionsgemisch wird wie üblich aufgearbeitet und das Rohprodukt an einer Kieselgelsäule mit Ethylacetat/Petrolether als Laufmittel chromatographiert: 2-(4-Nitrophenyl)-isothiazolidin-1,1-dioxid als gelblicher Feststoff; ESI 243.

Eine Lösung von 2.60 g (10.7 mmol) 2-(4-Nitrophenyl)-isothiazolidin-1,1-dioxid in 50 ml Tetrahydrofuran wird mit 1.0 g wasserfeuchtem Raney-Nickel versetzt und bei Raumtemperatur hydriert. Der Katalysator wird abfiltriert und das Filtrat eingedampft: 2-(4-Aminophenyl)-isothiazolidin-1,1-dioxid als bräunlicher Feststoff; ESI 213.

Eine Lösung von 212 mg (1.00 mmol) 2-(4-Aminophenyl)-isothiazolidin-1,1-dioxid, 276 mg (1.00 mmol) 2-[3-(5-Methyl-[1,2,4] oxadiazol-3-yl)-phenoxy]-pentansäure, 192 mg (1.00 mmol) N-(3-Dimethylaminopropyl)-*N'*-ethylcarbodiimidhydrochlorid (DAPECI) und 135 mg (1.00 mmol) Hydroxybenztriazolhydrat (HOBt) in 1 ml DMF wird mit 101 *µ*l (1.00 mmol) 4-Methylmorpholin versetzt und 24 Stunden bei Raumtemperatur gerührt. Das Reaktionsgemisch wird auf Wasser gegeben und der Niederschlag abfiltriert: 2-[3-(5-Methyl-[1,2,4]oxadiazol-3-yl)-phenoxy]-*N-*[4-(isothiazolidin-1,1-dioxid-2-yl)-phenyl] -valeriansäureamid als farbloser Feststoff; ESI 471.

Eine Lösung von 200 mg (0.425 mmol) 2-[3-(5-Methyl-[1,2,4]oxadiazol-3-yl)-phenoxy]-*N*-[4-(isothiazolidin-1, 1-dioxid-2-yl)-phenyl]-valeriansäureamid in 10 ml Methanol wird mit 1.5 g wasserfeuchtem Raney-Nickel, 0.5 ml Eisessig und 0.5 ml Wasser versetzt und hydriert. Das Reaktionsgemisch wird filtriert und das Filtrat eingedampft: 2-(3-Amidino-phenoxy)-*N*-[4-(isothiazolidin-1,1-dioxid-2-yl)-phenyl]-valeriansäureamid, Acetat ("AA") als farbloser Feststoff; ESI 431.

Analog erhält man die nachstehenden Verbindungen
2-(3-Amidino-phenoxy)-*N*-[4-(isothiazolidin-1,1-dioxid-2-yl)-3-methylphenyl]-valeriansäureamid, Acetat, ESI 335;
2-(3-Amidino-phenoxy)-*N*-[4-(isothiazolidin-1,1-dioxid-2-yl)-phenyl]-3-phenyl-propionamid.

### Beispiel 2

Die Herstellung von 2-(3-Aminomethyf-phenylamino)-*N-*[4-(isothiazolidin-1,1-dioxid-2-yl)-3-methyl-phenyl]-2-phenyl-acetamid erfolgt wie in nachstehendem Schema angegeben:

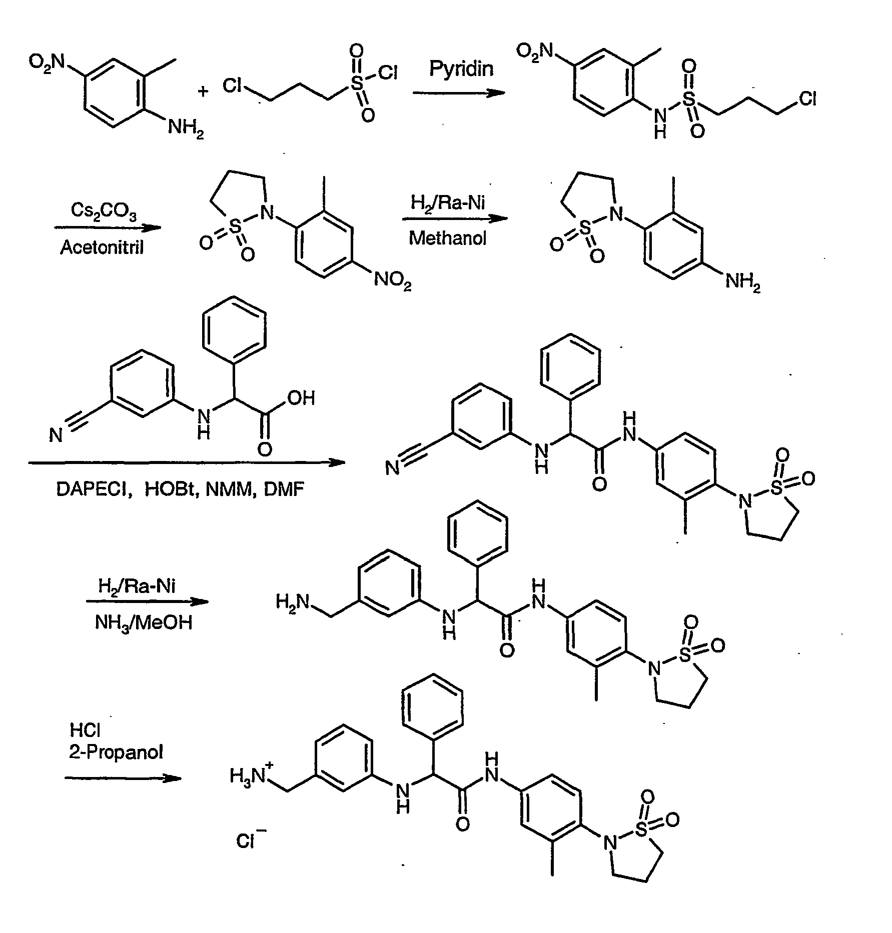

Eine Lösung von 5.51 g (36.2 mmol) 2-Methyl-4-nitroanilin und 6.41 g (36.2 mmol) 3-Chlorpropansulfonylchlorid in 20 ml Pyridin wird über Nacht bei Raumtemperatur gerührt. Dann wird das Reaktionsgemisch auf Eis gegossen. Der dabei ausgefallene Niederschlag wird abgesaugt und getrocknet: 3-Chlorpropan-1-sulfonsäure-(2-methyl-4-nitrophenyl)-amid als gelblicher Feststoff; ESI 293.

Eine Lösung von 6.50 g (22.2 mmol) 3-Chlorpropan-1-sulfonsäure-(2-methyl-4-nitrophenyl)-amid in 150 ml Acetonitril wird mit 11.1 g Caesiumcarbonat (34.0 mmol) versetzt und 18 Stunden bei 70°C gerührt. Das Reaktionsgemisch wird wie üblich aufgearbeitet und das Rohprodukt an einer Kieselgelsäule mit Ethylacetat/Petrolether als Laufmittel chromatographiert: 2-(2-Methyl-4-nitrophenyl)-isothiazolidin-1,1-dioxid als gelblicher Feststoff; ESI 257.

Eine Lösung von 2.50 g (9.76 mmol) 2-(2-Methyl-4-nitrophenyl)-isothiazolidin-1,1-dioxid in 50 ml Tetrahydrofuran wird mit 1.0 g wasserfeuchtem Raney-Nickel versetzt und bei Raumtemperatur hydriert. Der Katalysator wird abfiltriert und das Filtrat eingedampft: 2-(2-Methyl-4-aminophenyl)-isothiazolidin-1,1-dioxid als gelblicher Feststoff; ES I 227.

Eine Lösung von 113 mg (0.500 mmol) 2-(2-Methyl-4-aminophenyl)-isothiazolidin-1,1-dioxid, 126 mg (0.500 mmol) (3-Cyano-phenylamino)-phenylessigsäure, 96.0 mg (0.500 mmol) *N*-(3-Dimethylaminopropyl)-*N'-*ethylcarbodiimidhydrochlorid (DAPECI) und 68 mg (0.500 mmol) Hydroxybenztriazolhydrat (HOBt) in 1 ml DMF wird mit 51 *µ*l (0.500 mmol) 4-Methylmorpholin versetzt und 24 Stunden bei Raumtemperatur gerührt. Das Reaktionsgemisch wird auf Wasser gegeben und der Niederschlag abfiltriert: 2-(3-Cyano-phenylamino)-*N*-[4-(isothiazolidin-1,1-dioxid-2-yl)-3-methyl-phenyl]-2-phenyl-acetamid als farbloser Feststoff; ESI 461.

Eine Lösung von 200 mg (0.434 mmol) 2-(3-Cyano-phenylamino)-*N*-[4-(isothiazolidin-1,1-dioxid-2-yl)-3-methyl-phenyl]-2-phenyl-acetamid in 3 ml Methanol wird mit 200 mg wasserfeuchtem Raney-Nickel und 1.0 ml methanolischer Ammoniaklösung versetzt und bei 50°C hydriert. Das Reaktionsgemisch wird filtriert und das Filtrat eingedampft: 2-(3-Aminomethyl-phenylamino)-N [4-(isothiazolidin-1,1-dioxid-2-yl)-3-methyl-phenyl]-2-phenyl-acetamid als farbloser Feststoff; ESI 465.

137 mg (0.295 mmol) 2-(3-Aminomethyl-phenylamino)-*N*-[4-(isothiazolidin-1,1-dioxid-2-yl)-3-methyl-phenyl]-2-phenyl-acetamid wird in 2.95 ml (0.295 mmol) 0.1 N HCl in 2-Propanol gelöst und 2 Stunden bei Raumtemperatur stehen gelassen. Das Reaktionsgemisch wird eingedampft und anschließend lyophilisiert: 2-(3-Aminomethylphenylamino)-*N*-[4-(isothiazolidin-1,1-dioxid-2-yl)-3-methyl-phenyl] -2-phenyl-acetamid, Hydrochlorid ("BB") als farbloser Feststoff; ESI 465.

Analog erhält man die nachstehenden Verbindungen
2-(3-Aminomethyl-phenylamino)-*N-*[4-(isothiazolidin-1,1-dioxid-2-yl)-phenyl]-2-phenyl-acetamid, Hydrochlorid, ESI 451;
2-(3-Aminomethyl-phenylamino)-*N*-[3-chlor-4-(isothiazolidin-1,1-dioxid-2-yl)-phenyl]-2-phenyl-acetamid,
2-(3-Aminomethyl-phenylamino)-*N-*[3-chlor-4-(isothiazolidin-1,1-dioxid-2-yl)-phenyl]-2-(2-fluorphenyl)-acetamid,
2-(3-Aminomethyl-phenylamino)-*N*-[3-trifluormethyl-4-(isothiazolidin-1,1-dioxid-2-yl)-phenyl]-2-phenyl-acetamid,
2-(3-Aminomethyl-phenylamino)-*N*-[3-trifluormethyl-4-(isothiazolidin-1,1-dioxid-2-yl)-phenyl]-2-(2-fluorphenyl)-acetamid,
2-(3-Aminomethyl-phenylamino)-*N*-[4-(isothiazolidin-1,1-dioxid-2-yl)-3-methyl-phenyl]-3-phenyl-propionamid.

### Pharmakologische Daten

**Tabelle 1 Affinität zu Rezeptoren**

| Verbindung Nr. | FXa-IC₅₀ [M] | TF/FVIIa-IC₅₀ [M] |
|---|---|---|
| "AA" | 3.5 x 10⁻⁷ | 4.5 x 10⁻⁷ |
| "BB" | 4.9 x 10⁻⁷ | 8.7 x 10⁻⁷ |
| | | |

### Beispiel 3

Analog Beispiel 1 erhält man die nachstehende Verbindung
1-[1-(3-Amidino-phenyl)-piperidin-2-yl]-*N-*[4-(isothiazolidin-1,1-dioxid-2-yl)-phenyl]-carbonsäureamid

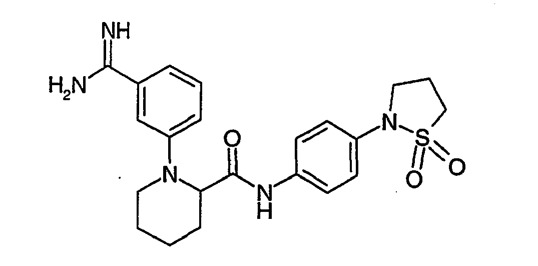

### Beispiel 4

Analog Beispiel 2 erhält man die nachstehenden Verbindungen
1-[1-(3-Aminomethyl-phenyl)-piperidin-2-yl]-*N*-[4-(isothiazolidin-1,1-dioxid-2-yl)-phenyl]-carbonsäureamid, ESI 429 und
1-[1-(3-Aminomethyl-phenyl)-piperidin-2-yl]-N-[4-(isothiazolidin-1, 1-dioxid-2-yl)-3-methyl-phenyl]-carbonsäureamid, ESI 443.

### Beispiel 5

Die Herstellung von 2-(1-Amino-isochinolin-7-yloxy)-*N*-[4-(isothiazolidin-1,1-dioxid-2-yl)-3-methyl-phenyl]-4-methyl-valeriansäureamid erfolgt wie in nachstehendem Schema angegeben:

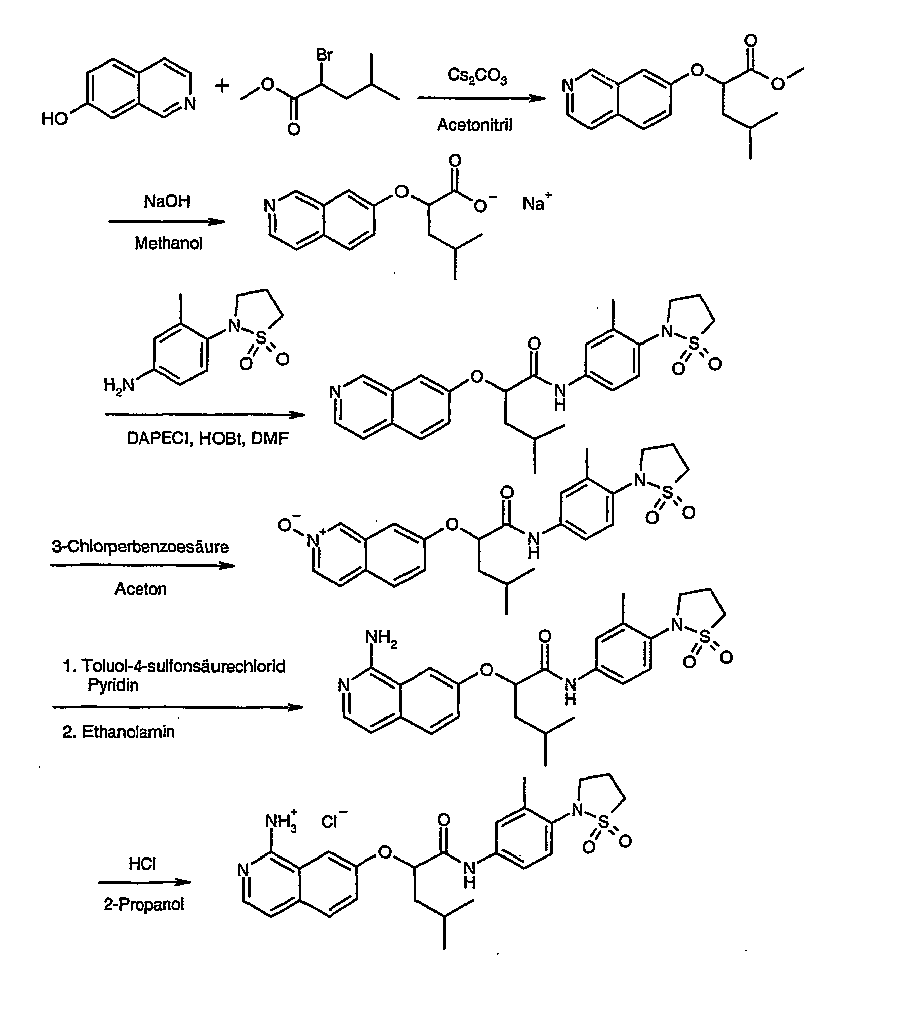

Analog erhält man die Verbindungen
(2S)-2-(1-Amino-isochinolin-7-yloxy)-*N*-[4-(isothiazolidin-1,1-dioxid-2-yl)-3-methyl-phenyl]-4-methyl-valeriansäureamid, ESI 483;
(2S)-2-(1-Amino-isochinolin-7-yloxy)-*N*-[4-(isothiazolidin-1,1-dioxid-2-yl)-phenyl]-4-methyl-valeriansäureamid, ESI 469.

Analog erhält man die nachstehenden Verbindungen
2-(1-Amino-isochinolin-7-yloxy)-*N*-[4-(isothiazolidin-1,1-dioxid-2-yl)-3-methyl-phenyl]-4-methyl-valeriansäureamid,
2-(1-Amino-isochinolin-7-yloxy)-*N*-[4-(isothiazolidin-1,1-dioxid-2-yl)-phenyl]-3-phenyl-propionamid.

### Beispiel 6

Die Herstellung von 2-[(4-Chlorphenyl)-ureido]-*N*-[4-(isothiazolidin-1,1-dioxid-2-yl)-3-methyl-phenyl]-2-phenyl-acetamid erfolgt wie in nachstehendem Schema angegeben:

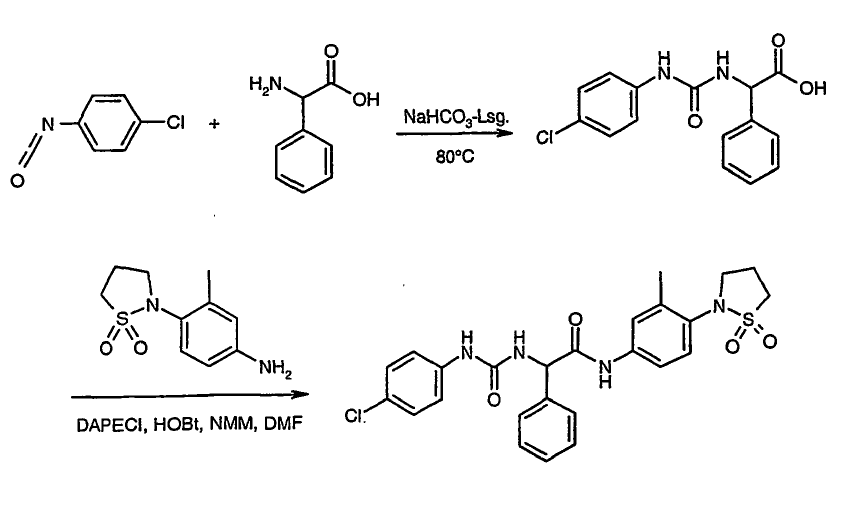

Analog erhält man ausgehend von (2R)-2-Phenylglycin die Verbindungen
(2R)-2-[(4-Chlorphenyl)-ureido]-*N*-[4-(isothiazolidin-1,1-dioxid-2-yl)-3-methyl-phenyl]-2-phenyl-acetamid, ESI 513 und
(2R)-2-[(4-Chlorphenyl)-ureido]-*N*-[4-(isothiazolidin-1,1-dioxid-2-yl)-phenyl]-2-phenyl-acetamid, ESI 513.

Analog erhält man die nachstehenden Verbindungen
2-[(4-Chlorphenyl)-ureido]-*N*-[4-(isothiazolidin-1,1-dioxid-2-yl)-3-methyl-phenyl]-3-phenyl-propionamid,
2-[(4-Chlorphenyl)-ureido]-*N*-[4-(isothiazolidin-1,1-dioxid-2-yl)-3-methyl-phenyl]-valeriansäureamid.

Die Herstellung von 2-[*N*-(4-Chlorphenyl)-carbamoyloxy]-*N*-[4-(isothiazolidin-1,1-dioxid-2-yl)-3-methyl-phenyl]-2-phenyl-acetamid erfolgt wie in nachstehendem Schema angegeben:

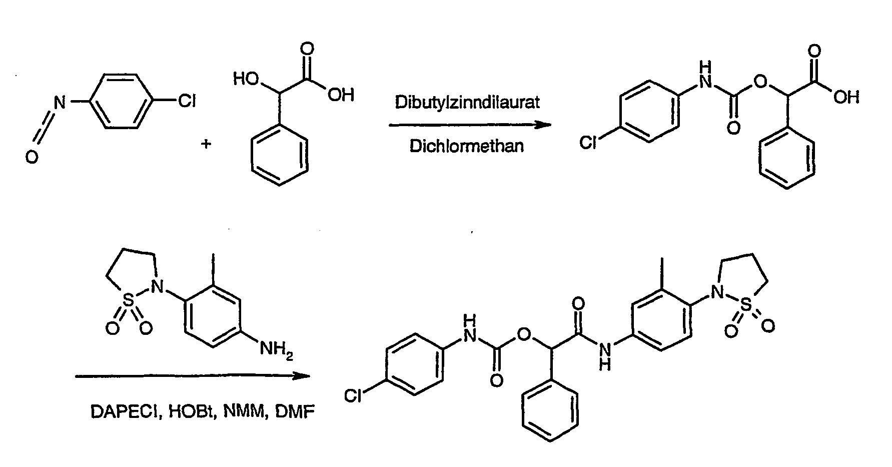

Analog erhält man ausgehend von (2R)-2-Hydroxy-phenylessigsäure die Verbindungen
(2R)-2-[*N*-(4-Chlorphenyl)-carbamoyloxy]-*N*-[4-(isothiazolidin-1,1- dioxid-2-yl)-3-methyl-phenyl]-2-phenyl-acetamid, ESI 514 und
(2R)-2-[*N*-(4-Chlorphenyl)-carbamoyloxy]-*N*-[4-(isothiazolidin-1,1-dioxid-2-yl)-phenyl]-2-phenyl-acetamid, ESI 500.

Analog erhält man die nachstehenden Verbindungen
2-[*N*-(4-Chlorphenyl)-carbamoyloxy]-*N*-[4-(isothiazolidin-1,1-dioxid-2-yl)-3-methyl-phenyl]-valeriansäureamid und
2-[*N-*(4-Chlorphenyl)-carbamoyloxy]-*N*-[4-(isothiazolidin-1,1-dioxid-2-yl)-3-methyl-phenyl]-3-phenyl-propionamid.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel 1, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel 1 werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel 1 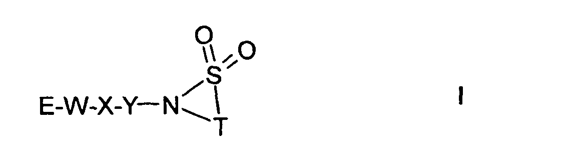 worin
E einfach durch R¹ substituiertes Phenyl oder Isochinolyl,
R¹ CN, Amidino, Hal, NH₂, CH₂NH₂ oder 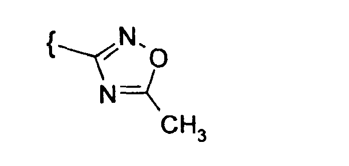
W OCHAr', OCHA, NHCHAr', NHCHA, NHCOOCHAr', NHCONHCHAr' oder Piperidin-1 ,2-diyl,
Ar' unsubstituiertes oder ein- oder zweifach durch Hal, A oder CF₃ substituiertes Phenyl,
A Alkyl mit 1, 2, 3, 4, 5, 6, 7 oder 8 C-Atomen,
X CONH,
Y Ar-diyl,
T (CH₂)₃.
Ar unsubstituiertes oder ein- oder zweifach durch Hal, A oder CF₃ substituiertes Phenyl,
Hal F, Cl, Br oder 1
bedeuten,
sowie ihre pharmazeutisch verwendbaren Salze, Solvate und Stereoisomere, einschließlich deren Mischungen in allen Verhältnissen.

2. Verbindungen nach Anspruch 1 ausgewählt aus der Gruppe
2-(3-Amidino-phenoxy)-*N*-[4-(isothiazolidin-1,1-dioxid-2-yl)-phenyl]-valeriansäureamid,
2-(3-Amidino-phenoxy)-*N*-[4-(isothiazolidin-1,1-dioxid-2-yl)-3-methylphenyl]-valeriansäureamid,
2-(3-Amidino-phenoxy)-*N*-[4-(isothiazolidin-1,1-dioxid-2-yl)-phenyl]-3-phenyl-propionamid,
2-(3-Aminomethyl-phenylamino)-*N*-[4-(isothiazolidin-1,1-dioxid-2-yl)-3-methyl-phenyl]-2-phenyl-acetamid,
2-(3-Aminomethyl-phenylamino)-*N*-[4-(isothiazolidin-1,1-dioxid-2-yl)-phenyl]-2-phenyl-acetamid, Hydrochlorid,
2-(3-Aminomethyl-phenylamino)-*N*-[3-chlor-4-(isothiazolidin-1,1- dioxid-2-yl)-phenyl]-2-phenyl-acetamid,
2-(3-Aminomethyl-phenylamino)-*N*-[3-chlor-4-(isothiazolidin-1,1-dioxid-2-yl)-phenyl]-2-(2-fluorphenyl)-acetamid,
2-(3-Aminomethyl-phenylamino)-*N*-[3-trifluormethyl-4-(isothiazolidin-1,1-dioxid-2-yl)-phenyl]-2-phenyl-acetamid,
2-(3-Aminomethyl-phenylamino)-*N*-[3-trifluormethyl-4-(isothiazolidin-1,1-dioxid-2-yl)-phenyl]-2-(2-fluorphenyl)-acetamid,
2-(3-Aminomethyl-phenylamino)-*N*-[4-(isothiazolidin-1,1-dioxid-2-yl)-3-methyl-phenyl]-3-phenyl-propionamid,
1-[1-(3-Amidino-phenyl)-piperidin-2-yl]-*N*-[4-(isothiazolidin-1,1-dioxid-2-yl)-phenyl]-carbonsäureamid,
1-[1-(3-Aminomethyl-phenyl)-piperidin-2-yl]-*N*-[4-(isothiazolidin-1,1-dioxid-2-yl)-phenyl]-carbonsäureamid,
2-(1-Amino-isochinolin-7-yloxy)-*N*-[4-(isothiazolidin-1,1-dioxid-2-yl)-phenyl]-valeriansäureamid,
2-(1-Amino-isochinolin-7-yloxy)-*N*-[4-(isothiazolidin-1,1-dioxid-2-yl)-3-methyl-phenyl]-4-methyl-valeriansäureamid,
2-(1-Amino-isochinolin-7-yloxy)-*N*-[4-(isothiazolidin-1,1-dioxid-2-yl)-phenyl]-3-phenyl-propionamid,
2-[(4-Chlorphenyl)-ureido]-*N*-[4-(isothiazolidin-1,1-dioxid-2-yl)-3-methyl-phenyl]-2-phenyl-acetamid,
2-[(4-Chlorphenyl)-ureido]-*N*-[4-(isothiazolidin-1,1-dioxid-2-yl)-3-methyl-phenyl]-3-phenyl-propionamid,
2-[(4-Chlorphenyl)-ureido]-*N*-[4-(isothiazolidin-1,1-dioxid-2-yl)-3-methyl-phenyl]-valeriansäureamid,
2-[*N*-(4-Chlorphenyl)-carbamoyloxy]-*N*-[4-(isothiazotidin-1,1-dioxid-2-yl)-3-methyl-phenyl]-2-phenyl-acetamid,
2-[*N*-(4-Chlorphenyl)-carbamoyloxy]-*N*-[4-(isothiazolidin-1,1-dioxid-2-yl)-3-methyl-phenyl]-valeriansäureamid,
2-[*N*-(4-Chlorphenyl)-carbamoyloxy]-*N*-[4-(isothiazolidin-1,1-dioxid-2-yl)-3-methyl-phenyl]-3-phenyl-propionamid,
sowie ihre pharmazeutisch verwendbaren Salze, Solvate und Stereoisomere, einschließlich deren Mischungen in allen Verhältnissen.

3. Verfahren zur Herstellung von Verbindungen der Formel I gemäß Anspruch 1 sowie ihrer pharmazeutisch verwendbaren Salze, Solvate und Stereoisomere, **dadurch gekennzeichnet, daß** man
a) sie aus einem ihrer funktionellen Derivate durch Behandeln mit einem solvolysierenden und/oder hydrogenolysierenden Mittel in Freiheit setzt, indem man
i) eine Amidinogruppe aus ihrem Oxadiazolderivat oder Oxazolidinonderivat durch Hydrogenolyse oder Solvolyse freisetzt,
ii) eine konventionelle Aminoschutzgruppe durch Behandeln mit einem solvolysierenden oder hydrogenolysierenden Mittel durch Wasserstoff ersetzt oder eine durch eine konventionelle Schutzgruppe geschützte Aminogruppe in Freiheit setzt,
b) einen Rest E in einen anderen Rest E umwandelt, indem man
i) eine Cyangruppe zu einer Amidinogruppe umsetzt,
ii) eine Amidgruppe zu einer Aminoalkylgruppe reduziert,
iii) eine Cyangruppe zu einer Aminoalkylgruppe reduziert,
c) zur Herstellung einer Verbindung der Formel I,
worin X CONH bedeutet,
eine Verbindung der Formel IV
E-W-CO-L IV
worin
L Cl, Br, I oder eine freie oder reaktionsfähig funktionell abgewandelte OH-Gruppe bedeutet bedeutet
und E und W die in Anspruch 1 angegebenen Bedeutungen haben, mit der Maßgabe, daß falls eine weitere OH- und/oder Aminogruppe vorliegt, diese geschützt ist,
mit einer Verbindung der Formel V 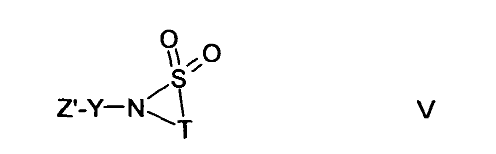 worin
Z' NH₂ bedeutet
und Y und T die in Anspruch 1 angegebenen Bedeutungen haben, umsetzt,
und anschließend gegebenenfalls eine Schutzgruppe abspaltet,
und/oder
eine Base oder Säure der Formel l in eines ihrer Salze umwandelt.

4. Arzneimittel, enthaltend mindestens eine Verbindung der Formel 1 gemäß Anspruch 1 oder 2 und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

5. Arzneimittel enthaltend mindestens eine Verbindung der Formel I gemäß Anspruch 1 oder 2 und/oder ihre pharmazeutisch verwendbaren Salze, Solvate und Stereoisomere, einschließlich deren Mischungen in allen Verhältnissen, und mindestens einen weiteren Arzneimittelwirkstoff.

6. Verwendung von Verbindungen gemäß Anspruch 1 oder 2 und/oder ihre physiologisch unbedenklichen Salze und Solvate zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen.

7. Set (Kit), bestehend aus getrennten Packungen von
(a) einer wirksamen Menge an einer Verbindung der Formel 1 gemäß Anspruch 1 oder 2und/oder ihrer pharmazeutisch verwendbaren Salze, Solvate und Stereoisomere, einschließlich deren Mischungen in allen Verhältnissen,
und
(b) einer wirksamen Menge eines weiteren Arzneimittelswirkstoffs.

8. Verwendung von Verbindungen der Formel I gemäß Anspruch 1 oder 2 und/oder ihrer pharmazeutisch verwendbaren Salze, Solvate und Stereoisomere, einschließlich deren Mischungen in allen Verhältnissen,
zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen,
in Kombination mit mindestens einem weiteren Arzneimittelwirkstoff.

9. Zwischenverbindungen der Formel I-I 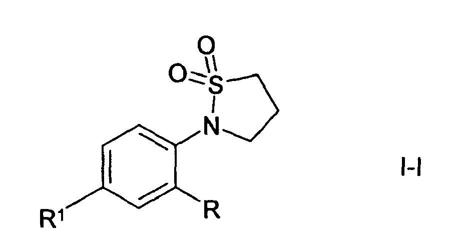 worin
R¹ NO₂ oder NH₂,
R Methyl, Chlor oder Trifluormethyl
bedeuten,
sowie deren Salze.

## Claims

1. Compounds of the formula I 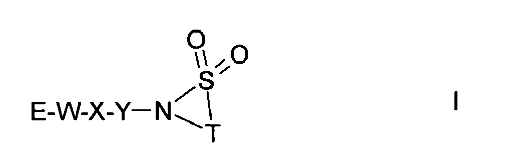 in which
E denotes phenyl or isoquinolyl, each of which is monosubstituted by R¹,
R¹ denotes CN, amidino, Hal, NH₂, CH₂NH₂ or 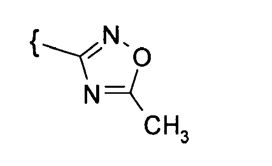
W denotes OCHAr', OCHA, NHCHAr', NHCHA, NHCOOCHAr', NHCONHCHAr' or piperidine-1 ,2-diyl,
Ar' denotes phenyl which is unsubstituted or mono- or disubstituted by Hal, A or CF₃,
A denotes alkyl having 1, 2, 3, 4, 5, 6, 7 or 8 C atoms,
X denotes CONH,
Y denotes Ar-diyl,
T denotes (CH₂)₃,
Ar denotes phenyl which is unsubstituted or mono- or disubstituted by Hal, A or CF₃,
Hal denotes F, Cl, Br or I,
and pharmaceutically usable salts, solvates and stereoisomers thereof, including mixtures thereof in all ratios.

2. Compounds according to Claim 1 selected from the group
2-(3-amidinophenoxy)-*N*-[4-(1,1-dioxoisothiazolidin-2-yl)phenyl]-valeramide,
2-(3-amidinophenoxy)-*N*-[4-(1,1-dioxoisothiazolidin-2-yl)-3-methylphenyl]valeramide,
2-(3-amidinophenoxy)-*N*-[4-(1,1-dioxoisothiazolidin-2-yl)phenyl]-3-phenylpropionamide,
2-(3-aminomethylphenylamino)-*N*-[4-(1,1-dioxoisothiazolidin-2-yl)-3-methylphenyl]-2-phenylacetamide,
2-(3-aminomethylphenylamino)-*N*-[4-(1, 1-dioxoisothiazolidin-2-yl)-phenyl]-2-phenylacetamide, hydrochloride,
2-(3-aminomethylphenylamino)-*N*-[3-chloro-4-(1,1-dioxoisothiazolidin-2-yl)phenyl]-2-phenylacetamide,
2-(3-aminomethylphenylamino)-*N*-[3-chloro-4-(1,1-dioxoisothiazolidin-2-yl)phenyl]-2-(2-fluorophenyl)acetamide,
2-(3-aminomethylphenylamino)-*N*-[3-trifluoromethyl-4-(1,1-dioxoisothiazolidin-2-yl)phenyl]-2-phenylacetamide,
2-(3-aminomethylphenylamino)-*N*-[3-trifluoromethyl-4-(1,1-dioxoisothiazolidin-2-yl)phenyl]-2-(2-fluorophenyl)acetamide,
2-(3-aminomethylphenylamino)-*N*-[4-(1,1-dioxoisothiazolidin-2-yl)-3-methylphenyl]-3-phenylpropionamide,
1-[1-(3-amidinophenyl)piperidin-2-yl]-*N*-[4-(1,1-dioxoisothiazolidin-2-yl)phenyl]carboxamide,
1-[1-(3-aminomethylphenyl)piperidin-2-yl]-*N*-[4-(1,1-dioxoisothiazolidin-2-yl)phenyl]carboxamide,
2-(1-aminoisoquinolin-7-yloxy)-*N*-[4-(1,1-dioxoisothiazolidin-2-yl)-phenyl]valeramide,
2-(1-aminoisoquinolin-7-yloxy)-*N*-[4-(1,1-dioxoisothiazolidin-2-yl)-3-methylphenyl]-4-methylvaleramide,
2-(1-aminoisoquinolin-7-yloxy)-*N*-[4-(1,1-dioxoisothiazolidin-2-yl)-phenyl]-3-phenylpropionamide,
2-[(4-chlorophenyl)ureido]-*N*-[4-(1,1-dioxoisothiazolidin-2-yl)-3-methylphenyl]-2-phenylacetamide,
2-[(4-chlorophenyl)ureido]-*N*-[4-(1,1-dioxoisothiazolidin-2-yl)-3-methylphenyl]-3-phenylpropionamide,
2-[(4-chlorophenyl)ureido]-*N*-[4-(1,1-dioxoisothiazolidin-2-yl)-3-methylphenyl]valeramide,
2-[*N*-(4-chlorophenyl)carbamoyloxy]-*N*-[4-(1,1-dioxoisothiazolidin-2-yl) -3-methylphenyl] -2-phenylacetamide,
2-[*N*-(4-chlorophenyl)carbamoyloxy]-*N*-[4-(1,1-dioxoisothiazolidin-2-yl)-3-methylphenyl]valeramide,
2-[*N*-(4-chlorophenyl)carbamoyloxy]-*N*-[4-(1,1-dioxoisothiazolidin-2-yl)-3-methylphenyl]-3-phenylpropionamide,
and pharmaceutically usable salts, solvates and stereoisomers thereof, including mixtures thereof in all ratios.

3. Process for the preparation of compounds of the formula I according to Claim 1 and pharmaceutically usable salts, solvates and stereoisomers thereof, **characterised in that**
a) they are liberated from one of their functional derivatives by treatment with a solvolysing and/or hydrogenolysing agent by
i) liberating an amidino group from its oxadiazole derivative or oxazolidinone derivative by hydrogenolysis or solvolysis,
ii) replacing a conventional amino-protecting group with hydrogen by treatment with a solvolysing or hydrogenolysing agent, or liberating an amino group protected by a conventional protecting group,
b) a radical E is converted into another radical E by
i) converting a cyano group into an amidino group,
ii) reducing an amide group to an aminoalkyl group,
iii) reducing a cyano group to an aminoalkyl group,
c) for the preparation of a compound of the formula I
in which X denotes CONH,
a compound of the formula IV
E-W-CO-L IV
in which
L denotes Cl, Br, I or a free or reactively functionally modified OH group,
and E and W have the meanings indicated in Claim 1,
with the proviso that any further OH and/or amino group present is protected,
is reacted with a compound of the formula V 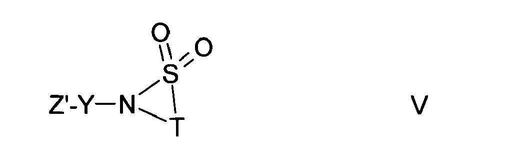 in which
Z' denotes NH₂,
and Y and T have the meanings indicated in Claim 1,
and a protecting group is optionally subsequently removed,
and/or
a base or acid of the formula I is converted into one of its salts.

4. Medicaments comprising at least one compound of the formula I according to Claim 1 or 2 and/or pharmaceutically usable salts, solvates and stereoisomers thereof, including mixtures thereof in all ratios, and optionally excipients and/or adjuvants.

5. Medicaments comprising at least one compound of the formula I according to Claim 1 or 2 and/or pharmaceutically usable salts, solvates and stereoisomers thereof, including mixtures thereof in all ratios, and at least one further medicament active ingredient.

6. Use of compounds according to Claim 1 or 2 and/or physiologically acceptable salts and solvates thereof for the preparation of a medicament for the treatment of thromboses, myocardial infarction, arteriosclerosis, inflammation, apoplexy, angina pectoris, restenosis after angioplasty, claudicatio intermittens, migraine, tumours, tumour diseases and/or tumour metastases.

7. Set (kit) consisting of separate packs of
(a) an effective amount of a compound of the formula I according to Claim 1 or 2 and/or pharmaceutically usable salts, solvates and stereoisomers thereof, including mixtures thereof in all ratios,
and
(b) an effective amount of a further medicament active ingredient.

8. Use of compounds of the formula I according to Claim 1 or 2 and/or pharmaceutically usable salts, solvates and stereoisomers thereof, including mixtures thereof in all ratios,
for the preparation of a medicament for the treatment of thromboses, myocardial infarction, arteriosclerosis, inflammation, apoplexy, angina pectoris, restenosis after angioplasty, claudicatio intermittens, migraine, tumours, tumour diseases and/or tumour metastases,
in combination with at least one further medicament active ingredient.

9. Intermediates of the formula I-I 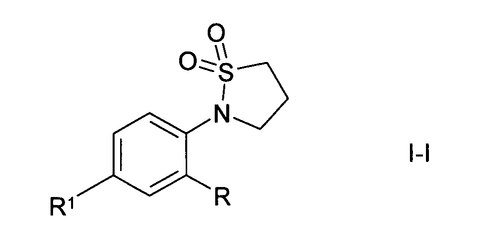 in which
R¹ denotes NO₂ or NH₂,
R denotes methyl, chlorine or trifluoromethyl,
and salts thereof.

## Revendications

1. Composés de la formule 1 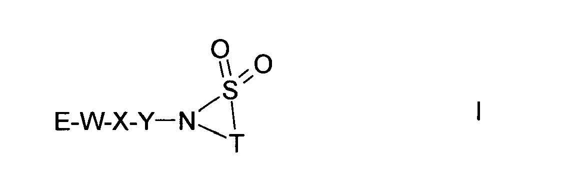 dans laquelle
E représente phényle ou isoquinolyle dont chacun est monosubstitué par R¹,
R¹ représente CN, amidino, Hal, NH₂, CH₂NH₂ ou 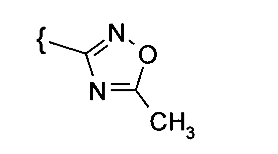
W représente OCHAr', OCHA, NHCHAr', NHCHA, NHCOOCHAr', NHCONHCHAr' ou pipéridine-1 ,2-diyle
Ar' représente phényle qui est non substitué ou mono- ou disubstitué par Hal, A ou CF₃,
A représente alkyle comportant 1, 2, 3, 4, 5, 6, 7 ou 8 atomes de C,
X représente CONH,
Y représente Ar-diyle,
T représente (CH₂)₃,
Ar représente phényle qui est non substitué ou mono- ou disubstitué par Hal, A ou CF₃,
Hal représente F, Cl, Br ou l,
et leurs sels, solvates et stéréo-isomères utilisables pharmaceutiquement, y compris des mélanges afférents selon tous rapports.

2. Composés selon la revendication 1, choisis parmi le groupe
2-(3-amidinophénoxy)-*N*-[4-(1,1-dioxoisothiazolidine-2-yl)phényl]-valéramide,
2-(3-amidinophénoxy)-*N*-[4-(1,1-dioxoisothiazolidine-2-yl)-3-méthylphényl]valéramide,
2-(3-amidinophénoxy)-*N*-[4-(1,1-dioxoisothiazolidine-2-yl)phényl]-3-phénylpropionamide,
2-(3-aminométhylphénylamino)-*N*-[4-(1 ,1-dioxoisothiazolidine-2-yl)-3-méthylphényl]-2-phénylacétamide,
hydrochlorure de 2-(3-aminométhylphénylamino)-*N*-[4-(1,1-dioxoisothiazolidine-2-yl)-phényl]-2-phénylacétamide,
2-(3-aminométhylphénylamino)-*N*-[3-chloro-4-(1,1-dioxoisothiazolidine-2-yl)phényl]-2-phénylacétamide,
2-(3-aminométhylphénylamino)-*N*-[3-chloro-4-(1,1-dioxoisothiazolidine-2-yl)phényl]-2-(2-fluorophényl)acétamide,
2-(3-aminométhylphénylamino)-*N*-[3-trifluorométhyl-4-(1,1-dioxoisothiazolidine-2-yl)phényl]-2-phénylacétamide,
2-(3-aminométhylphénylamino)-*N*-[3-trifluorométhyl-4-(1,1-dioxoisothiazolidine-2-yl)phényl]-2-(2-fluorophényl)acétamide,
2-(3-aminométhylphénylamino)-*N*-[4-(1,1-dioxoisothiazolidine-2-yl)-3-méthylphényl]-3-phénylpropionamide,
1-[1-(3-amidinophényl)pipéridine-2-yl]-*N*-[4-(1,1-dioxoisothiazolidine-2-yl)phényl]carboxamide,
1-[1-(3-aminométhylphényl)pipéridine-2-yl]-*N*-[4-(1,1-dioxoisothiazolidine-2-yl)phényl]carboxamide,
2-(1-aminoisoquinoline-7-yloxy)-*N*-[4-(1,1-dioxoisothiazolidine-2-yl)-phényl]valéramide,
2-(1-aminoisoquinoline-7-yloxy)-*N*-[4-(1,1-dioxoisothiazolidine-2-yl)-3-méthylphényl]-4-méthylvaléramide,
2-(1-aminoisoquinoline-7-yloxy)-*N*-[4-(1,1-dioxoisothiazolidine-2-yl)-phényl]-3-phénylpropionamide,
2-[(4-chlorophényl)ureido]-*N*-[4-(1,1-dioxoisothiazolidine-2-yl)-3-méthylphényl]-2-phénylacétamide,
2-[(4-chlorophényl)ureido]-*N*-[4-(1,1-dioxoisothiazolidine-2-yl)-3-méthylphényl]-3-phénylpropionamide,
2-[(4-chlorophényl)ureido]-*N*-[4-(1,1-dioxoisothiazolidine-2-yl)-3-méthylphényl]valéramide,
2-[*N*-(4-chlorophényl)carbamoyloxy]-*N*-[4-(1,1-dioxoisothiazolidine-2-yl)-3-méthylphényl]-2-phénylacétamide,
2-[*N*-(4-chlorophényl)carbamoyloxy]-*N*-[4-(1,1-dioxoisothiazolidine-2-yl)-3-méthylphényl]valéramide,
2-[*N*-(4-chlorophényl)carbamoyloxy]-*N*-[4-(1,1-dioxoisothiazolidine-2-yl)-3-méthylphényl]-3-phénylpropionamide,
et leurs sels, solvates et stéréo-isomères utilisables pharmaceutiquement, y compris des mélanges afférents selon tous rapports.

3. Procédé pour la préparation de composés de la formule 1 selon la revendication 1 et de leurs sels, solvates et stéréo-isomères utilisables pharmaceutiquement, **caractérisé en ce que** :
a) ils sont libérés à partir de l'un de leurs dérivés fonctionnels par traitement à l'aide d'un agent solvolysant et/ou hydrogénolysant en
i) libérant un groupe amidino à partir de son dérivé oxadiazole ou de son dérivé oxazolydinone par hydrogénolyse ou solvolyse,
ii) ii) remplaçant un groupe de protection amino classique par un hydrogène au moyen d'un traitement à l'aide d'un agent solvolysant ou hydrogénolysant, ou en libérant un groupe amino protégé au moyen d'un groupe de protection classique,
b) un radical E est converti selon un autre radical E en
i) convertissant un groupe cyano selon un groupe amidino,
ii) réduisant un groupe amide selon un groupe aminoalkyle,
iii) réduisant un groupe cyano selon un groupe aminoalkyle,
c) pour la préparation d'un composé de la formule I dans laquelle X représente CONH,
un composé de la formule IV
E-W-CO-L IV
dans laquelle
L représente Cl, Br, I ou un groupe OH libre ou fonctionnellement modifié de façon réactive,
et E et W présentent les significations indiquées selon la revendication 1,
étant entendu qu'un quelconque autre groupe OH et/ou amino présent est protégé
est amené à réagir avec un composé de la formule V 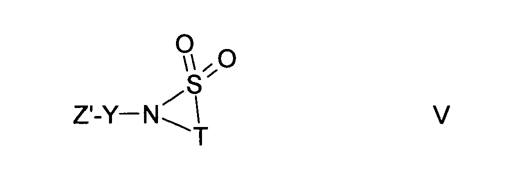 dans laquelle
Z' représente NH₂,
et Y et T présentent les significations indiquées selon la revendication 1,
et un groupe de protection est ensuite en option enlevé,
et/ou
une base ou un acide de la formule I est converti(e) selon l'un de ses sels.

4. Médicaments comprenant au moins un composé de la formule 1 selon la revendication 1 ou 2 et/ou leurs sels, solvates et stéréo-isomères utilisables pharmaceutiquement, y compris des mélanges afférents selon tous rapports et en option, des excipients et/ou des adjuvants.

5. Médicaments comprenant au moins un composé de la formule l selon la revendication 1 ou 2 et/ou leurs sels, solvates et stéréo-isomères utilisables pharmaceutiquement, y compris des mélanges afférents selon tous rapports et au moins un autre ingrédient actif de médicament.

6. Utilisation de composés selon la revendication 1 ou 2 et/ou de leurs sels et solvates acceptables physiologiquement pour la préparation d'un médicament pour le traitement des thromboses, de l'infarctus du myocarde, de l'artériosclérose, des inflammations, de l'apoplexie, de l'angine de poitrine, de la sténose après angioplastie, de la claudication intermittente, de la migraine, des tumeurs, des maladies tumorales et/ou des métastases tumorales.

7. Jeu (kit) constitué par des groupements séparés de
(a) une quantité efficace d'un composé de la formule I selon la revendication 1 ou 2 et/ou de leurs sels, solvates et stéréo-isomères utilisables pharmaceutiquement, y compris des mélanges afférents selon tous rapports
et
(b) une quantité efficace d'un autre ingrédient actif de médicament.

8. Utilisation de composés de la formule l selon la revendication 1 ou 2 et/ou de leurs sels, solvates et stéréo-isomères utilisables pharmaceutiquement, y compris des mélanges afférents selon tous rapports pour la préparation d'un médicament pour le traitement des thromboses, de l'infarctus du myocarde, de l'artériosclérose, des inflammations, de l'apoplexie, de l'angine de poitrine, de la sténose après angioplastie, de la claudication intermittente, de la migraine, des tumeurs, des maladies tumorales et/ou des métastases tumorales,
en combinaison avec au moins un autre ingrédient actif de médicament.

9. Produits intermédiaires de la formule I-I 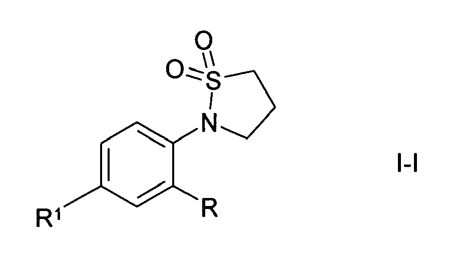 dans laquelle
R¹ représente NO₂ ou NH₂,
R représente méthyle, chlore ou trifluorométhyle,
et leurs sels.
